(19) Europäisches Patentamt
European Patent Office
Office européen des brevets

(11) **EP 3 081 652 B1**

(12) **EUROPÄISCHE PATENTSCHRIFT**

(45) Veröffentlichungstag und Bekanntmachung des Hinweises auf die Patenterteilung:
**28.06.2017 Patentblatt 2017/26**

(51) Int Cl.:
**C12Q 1/18** $^{(2006.01)}$     **G01N 1/00** $^{(2006.01)}$
**G01N 30/72** $^{(2006.01)}$

(21) Anmeldenummer: **15163322.9**

(22) Anmeldetag: **13.04.2015**

(54) **MASSENSPEKTROMETRISCHER SCHNELLTEST VON RESISTENZEN**

RAPID TESTING OF RESISTANCES BY MEANS OF MASS SPECTROMETRY

TEST RAPIDE PAR SPECTROMÉTRIE DE MASSE DE RÉSISTANCES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**19.10.2016 Patentblatt 2016/42**

(73) Patentinhaber: **Bruker Daltonik GmbH**
**28359 Bremen (DE)**

(72) Erfinder:
• **Sparbier, Katrin**
**28357 Bremen (DE)**
• **Wegemann, Beatrix**
**28207 Bremen (DE)**

(56) Entgegenhaltungen:
**EP-A1- 2 801 825     EP-A1- 2 806 275**
**WO-A1-2009/065580     WO-A1-2011/154517**
**WO-A1-2012/023845     US-A1- 2014 306 104**

• **Kerstin Reinecke:**
**"Massenspektrometrie-basierte Hochdurchsatz-Protein-Liganden-Bindungsassays", , 1 January 2006 (2006-01-01), pages 1-213, XP055290395, Retrieved from the Internet: URL:http://hdl.handle.net/11858/00-001M-00 00-0014-07E4-6 [retrieved on 2016-07-21]**

**Beschreibung**

Die Erfindung betrifft die Bestimmung der Resistenz von Mikroben gegen Antibiotika, insbesondere solcher Mikroben, die beta-Lactamasen produzieren.

**Stand der Technik**

In der Publikation WO 2011154517 A1 (M. Kostrzewa, K. Michelmann, K. Sparbier) und den äquivalenten Publikationen DE 102010023452 B4 und US 20130095511 A1 wird ein Verfahren zur Bestimmung von mikrobiellen beta-Lactamase-Resistenzen vorgestellt, das auf der massenspektrometrischen Messung der enzymatischen Veränderung von beta-Lactam-Antibiotika oder strukturähnlicher Substrate durch mikrobielle beta-Lactamasen beruht. Es werden dazu beispielsweise Bakterien in Lösungen mit beta-Lactam-Antibiotika oder strukturähnlichen Substraten eingeführt und für etwa drei Stunden inkubiert. Die Bakterien werden dann bevorzugt durch Zentrifugieren oder Filtrieren abgesondert. Ein bis zwei Mikroliter der bakterienfreien Lösung werden anschließend auf einen massenspektrometrischen Probenträger gegeben, eingetrocknet, und mit einer Matrixlösung für die matrix-unterstützte Laserdesorption und -ionisation (MALDI) überschichtet. Die Messung in einem Flugzeitmassenspektrometer mit einer MALDI-Ionenquelle ergibt ein Massenspektrum, aus dem bestimmt werden kann, ob ein enzymatisch verändertes Antibiotikum oder Substrat vorliegt, insbesondere eine hydrolytische Spaltung des beta-LactamRings, wodurch die Resistenz der Mikroben angezeigt ist.

Ein ähnliches Verfahren ist in der Publikation WO 2012/023845 A1 (Luider et al.) vorgestellt, wobei generell die Veränderungen von Antibiotika durch mikrobielle Enzyme massenspektrometrisch festgestellt wird und im Speziellen die Inkubation auf einem massenspektrometrischen Probenräger durchgeführt wird. Die Dissertationsschrift von Kerstin Reinecke ("Massenspektrometrie-basierte Hochdurchsatz-Protein-Liganden-Bindungsassays", 2006) beschreibt zudem die Durchführung von Proteinbindungsassays auf massenspektrometrischen Probenträgern in einer feuchten Kammer.

Die in den beiden erstgenannten Schriften beschriebenen Verfahren weisen relativ viele Verfahrensschritte auf, insbesondere auch das mit zeitlichen Aufwand und oft manuellen Arbeitsschritten verbundene Zentrifugieren oder Filtrieren. Die bekannten Verfahren dauern in der Regel etwa drei Stunden. Es besteht weiterhin ein Bedarf für Verfahren zur Bestimmung von mikrobiellen Resistenzen, die weniger aufwendig, leichter automatisierbar und schneller sind und die einen hohen Probendurchsatz bei geringeren Probenmengen ermöglichen.

**Aufgabe der Erfindung**

Es ist die Aufgabe der Erfindung, Verfahren und Geräte bereitzustellen, mit denen eine mikrobielle Resistenz, die auf einer enzymatischen Veränderung von Antibiotika beruht, mit wenigen Verfahrensschritten und möglichst in weniger als einer Stunde massenspektrometrisch bestimmt werden kann.

**Kurze Beschreibung der Erfindung**

Die Erfindung stellt ein Verfahren bereit für die Bestimmung der Resistenz einer mikrobiellen Probe auf ein Antibiotikum oder auf ein Antibiotikum mit Inhibitor, wobei die mikrobielle Probe und das Antibiotikum oder die mikrobielle Probe und das Antibiotikum mit dem Inhibitor in einem Flüssigkeitsvolumen auf einem Probenort eines massenspektrometrischen Probenträgers zusammengeführt und inkubiert werden und durch eine massenspektrometrische Messung am Probenort untersucht wird, ob das Antibiotikum enzymatisch verändert wird, dadurch gekennzeichnet, dass - der Probenort vor dem Zusammenführen mit einer dosierten Menge des Antibiotikum oder des Antibiotikum mit Inhibitor zusammen mit mindestens einer Referenzsubstanz in Form einer trockenen Schicht belegt ist, und - eine Abnahme des Antibiotikums aus dem Vergleich mit dem Massensignal der mindestens einen Referenzsubstanz ermittelt wird und/oder die mindestens eine Referenzsubstanz zur Feststellung der quantitativen Änderung der Abbauprodukte des Antibiotikums verwendet wird. Der Massenbereich der massenspektrometrischen Messung ist bevorzugt kleiner als 1000 Dalton, insbesondere zwischen 250 und 750 Dalton.

Das Antibiotikum kann ein beta-lactam Antibiotikum aus der Gruppe der Penicilline (Benzylpenicilline, Oralpenicilline, Aminopenicilline, Isoxazylpenicilline, Acylaminopenicilline), der Cephalosporine, der Monobactame oder der Carbapeneme sein. Die mikrobielle Probe kann ebenfalls mit einem Kombinationspräparat aus einem beta-Lactam Antibiotikum und einem beta-Lactamase Inhibitor zusammengeführt werden, z.B. mit Clavulansäure in Kombination mit Amoxicillin, Tazobactam in Kombination mit Piperacillin oder Sulbactam mit einem beta-lactam Antibiotikum. Die mikrobielle Probe kann am Probenort auch mit mehr als einem Antibiotikum zusammengeführt werden.

Eine enzymatische Veränderung des Antibiotikums wird durch eine Abnahme des Antibiotikums oder durch eine Zunahme von einem oder mehreren Abbauprodukten oder durch beides aus den entsprechenden Massensignalen ermittelt. Die Abnahme des Antibiotikums wird insbesondere aus dem Vergleich mit dem Massensignal einer Referenzsubstanz ermittelt. Der Probenort ist dabei bereits vor dem Zusammenführen der mikrobielle Probe und des Antibiotikums mit der Referenzsubstanz belegt. Wie hier beschrieben kann die Referenzsubstanz aber auch erst nach dem Zusammenführen oder während der Präparation einer für die massenspektrometrische Messung geeigneten Probe, z.B. einer MALDI-Probe, zugeführt werden.

In einer bevorzugten Ausführungsform erfolgt die massenspektrometrische Messung in einem Massenspektrometer mit einer MALDI-Ionenquelle, insbesondere in einem Flugzeitmassenspektrometer, wobei der massenspektrometrische Probenträger eben ist und eine Vielzahl von Probenorten aufweist.

In einer weiteren Ausführungsform weist die mikrobielle Probe bevorzugt intakte Mikrobenzellen auf. Die Inkubationszeit der Mikrobenzellen in dem Flüssigkeitsvolumen beträgt in dem erfindungsgemäßen Verfahren beispielsweise weniger als eine Stunde, vorzugsweise nur etwa eine halbe Stunde. Die Inkubation in dem Flüssigkeitsvolumen erfolgt dabei bevorzugt bei Raumtemperatur. Die Anzahl der Mikrobenzellen, die auf einen Probenort aufgebracht werden, beträgt bevorzugt zwischen $10^3$ bis $10^7$. Bei der Verwendung von intakten Mikrobenzellen kann es von Vorteil sein, dass die Mikrobenzellen auch während der Präparation der für die massenspektrometrische Messung verwendeten Probe, insbesondere bei der Präparation einer MALDI-Probe, intakt bleiben, also nicht aufgeschlossen werden.

Die mikrobielle Probe kann aus intakten Mikrobenzellen bestehen, die einer Kolonie eines flächigen Nährmediums (z.B. einer Agar-Platte) entnommen werden oder die durch Zentrifugieren oder Filtrieren aus einem flüssigen Nährmedium abgetrennt werden. Das flüssige Nährmedium kann dabei insbesondere eine positive Blutkultur sein, wobei die Blutzellen bevorzugt vor dem Zentrifugieren oder Filtrieren zerstört werden. Die mikrobielle Probe kann aber auch eine nicht inkubierte Probe einer Körperflüssigkeit, wie z.B. eine Blutprobe, eine Urinprobe oder die Probe einer Spinalflüssigkeit, oder ein Abstrich sein. Ein Abstrich ist körpereigenes Untersuchungsmaterial aus der Oberfläche von Wunden oder Schleimhäuten (Mund, Nase, Harnröhre, Scheide, After).

In einer anderen Ausführungsform besteht die mikrobielle Probe aus aufgeschlossenen Mikrobenzellen (Zelllysat), wobei der Aufschluss der Mikroben auch am Probenort erfolgen kann. Für den Fall, dass intakte Mikrobenzellen der mikrobiellen Probe durch die Präparation einer MALDI-Probe am Probenort aufgeschlossen werden oder die mikrobielle Probe ein Zelllysat ist, können in der massenspektrometrischen Messung auch Massensignale von mikrobiellen Proteinen aufgenommen werden, mit denen die Mikroben zusätzlich taxonomisch identifiziert und/oder hinsichtlich ihres Resistenzverhaltens weiter charakterisiert werden können. Eine mikrobielle Probe kann auch dadurch gewonnen werden, dass Mikrobenzellen einer Kolonie auf einem flächigen Nährmedium mit einer Flüssigkeit ohne Antibiotikum beprobt werden und danach die Probeflüssigkeit als mikrobielle Probe mit dem Antibiotikum auf dem Probenträger zusammengeführt wird.

In einer weiteren Ausführungsform ist das Flüssigkeitsvolumen am Probenort kleiner als zehn Mikroliter, bevorzugt kleiner als fünf Mikroliter und insbesondere zwischen ein und drei Mikroliter. Nachdem die mikrobielle Probe und das Antibiotikum auf einem Probenort eines Probenträgers zusammengeführt worden sind, wird der Probenträger bevorzugt in einer feuchten Umgebung gehalten oder es wird dem Flüssigkeitsvolumen auf dem Probenort zusätzlich Flüssigkeit zugeführt, um ein Eintrocknen des Flüssigkeitsvolums für eine vorgegebene Inkubations- bzw. Reaktionszeit zu verhindern.

Die Erfindung stellt weiterhin einen Probenträger für eine massenspektrometrische Bestimmung der Resistenz einer mikrobiellen Probe bereit, der dadurch gekennzeichnet ist, dass der Probenträger eine Vielzahl von Probenorten aufweist und dass mindestens ein Probenort mit einer dosierten Menge des Antibiotikums oder des Antibiotikums mit Inhibitor zusammen mit mindestens einer Referenzsubstanz in Form einer trockenen Schicht belegt ist. Der Probenort kann mit mehreren Antibiotika belegt sein. Verschiedene Probenorte können dabei mit verschiedenen Antibiotika belegt sein. Zudem sind die Probenorte jeweils zusätzlich mit einem oder mehreren Referenzsubstanzen belegt.

Die massenspektrometrische Messung kann im Prinzip mit jedem Massenspektrometer vorgenommen werden, wie beispielsweise mit einem Quadrupol-Filter (insbesondere einem Triple-Quadrupole-Massenspektrometer), einem Flugzeitmassenspektrometer mit orthogonalem Ioneneinschuss (OTOF), einem Ionenzyklotronresonanz-Massenspektrometer (ICR-MS), einer elektrostatische Kingdon-Massenspektrometer oder einem Ionenfallen-Massenspektrometer. Es ist besonders günstig, dasselbe MALDI-Flugzeitmassenspektrometer zu benutzen, das derzeit routinemäßig auch für die Identifizierung von Mikroben, insbesondere von Bakterien, eingesetzt wird und das in der Regel im linearen Modus ohne Reflektor betrieben wird.

Die Erfindung bietet Vorteile, die darin bestehen, mit geringen Mengen an Mikroben und kleinen Flüssigkeitsvolumina auszukommen, nur wenige einfache Verfahrensschritte zu benötigen und insbesondere sehr schnell zu sein. Das erfindungsgemäße Verfahren ermöglicht insbesondere eine massenspektrometrische Bestimmung der Resistenz, ohne dass Mikrobenzellen aus einem Flüssigkeitsvolumen durch Zentrifugeren oder Filtrieren abgetrennt werden müssen.

## Beschreibung der Abbildungen

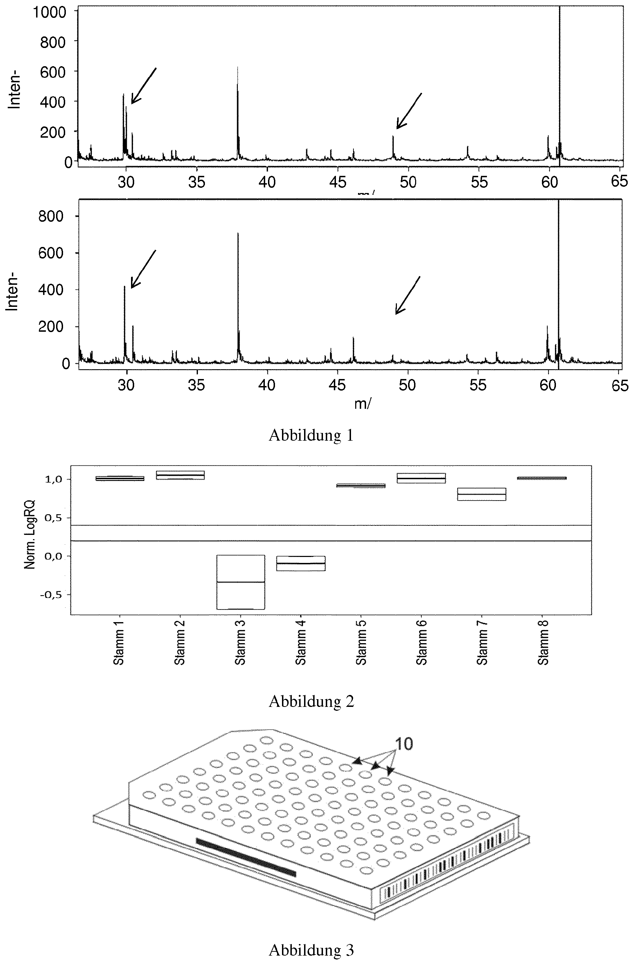

In Abbildung 1 zeigt das obere MALDI-Massenspektrum die Massensignale von nicht abgebautem Imipenem (markiert durch Pfeile) nach einer Inkubation mit einem beta-Lactamasenegativem Stamm. Das untere MALDI-Massenspektrum zeigt, dass die Massensignale des Imipenems nach einer halbstündigen Inkubation mit einem beta-Lactamase-positiven Stamm auf einem Probenträger verschwunden sind. Der senkrechte Strich gibt die Position der Referenzsubstanz an.

Die Abbildung 2 gibt ein "Boxplot-Diagramm" wieder, das die Quotienten *logRQ* von sechs beta-Lactamase-positiven Stämmen mit jeweils *logRQ* > 0,4 und zwei beta-Lactamase-negativen Stämme mit jeweils *logRQ* < 0,2 zeigt. Es sind Mediane und Streuungen aus mehreren Messungen gezeigt.

Abbildung 3 zeigt beispielhaft eine Probenträgerplatte in der Größe einer Mikrotiterplatte mit 96 Probenorten (10), die dosiert mit Antibiotika beschichtet sind. Für andere Typen von Massenspektrometern gibt es auch andere, meist kleinere Probenträgerplatten. Besonders günstig sind Probenträgerplatten, deren Probenorte (10) eine hydrophile Oberfläche haben, eingebettet in eine hydrophobe Umgebung. Aufgebrachte Flüssigkeiten fließen dann selbständig bis zum Rand der Probenorte.

## Detaillierte Beschreibung der Erfindung

Viele Arten von Mikroben, insbesondere Bakterien, Archäen und einzellige Pilze, lassen sich schnell und leicht massenspektrometrisch identifizieren, indem von einer Kolonie, die in üblicher Weise auf oder in einem Nährmedium gezüchtet wurde, kleine Mengen von Mikroben auf eine massenspektrometrischen Probenträgerplatte übertragen, dort mit einer Lösung einer Matrixsubstanz aufgeschlossen, getrocknet und mit Ionisierung durch matrixunterstützte Laserdesorption massenspektrometrisch vermessen werden. Aus dem Profil von jeweils etwa 40 bis 80 Proteinen, die typischerweise im Massenbereich zwischen 3000 und 20000 Dalton liegen, wird durch Ähnlichkeitsanalysen mit Tausenden von Referenzspektren die Identität der Mikroben festgestellt. Unter dem Begriff "Identifizierung" wird hier die taxonomische Klassifizierung, also die Bestimmung von Familie, Gattung (Genus), Art (Spezies) und gegebenenfalls Unterart (Subspezies) verstanden. Es gibt inzwischen medizin-rechtlich verwendbare Bibliotheken mit Referenzspektren von Tausenden von Mikrobenarten, darunter praktisch alle klinisch relevanten Spezies.

Im medizinischen Bereich tritt aber nicht nur das Problem einer schnellen Identifizierung, sondern auch das Problem der Erkennung von Resistenzen gegen die gebräuchlichen Antibiotika auf. Ohne Kenntnisse über die Resistenzen ist eine schnelle Bekämpfung oft nicht möglich. Es bedarf daher neben Verfahren für eine schnelle Identifizierung auch der Verfahren für eine schnelle Feststellung und Charakterisierung der Resistenzen von Mikroorganismen.

Seit den ersten Anwendungen des Penicillins, einem beta-Lactam-Antibiotikum, haben Bakterien in zunehmendem Maße verschiedenartige Resistenzen entwickelt. Eine Art der Resistenz von Bakterien gegenüber beta-Lactamen besteht in der Ausbildung von Enzymen (beta-Lactamasen), die den beta-Lactamring katalytisch durch Hydrolyse aufsprengen und damit unwirksam machen. Durch die katalytische Wirkung dieser Enzyme genügt eine geringe Menge an beta-Lactamasen, um große Mengen an beta-Lactam-Antibiotika zu zerstören. Zurzeit sind mehrere Hundert Varianten von beta-Lactamasen bekannt, die von verschiedenartigen Bakterien gebildet werden. Die zunächst durch Mutationen erzeugte genetische Information zur Synthese des Enzyms wird chromosomal oder plasmidal vererbt, die plasmidale Information kann auch durch verschiedene Mechanismen zwischen Bakterien ausgetauscht werden, sogar zwischen Bakterien verschiedener Arten ("Horizontalaustausch"), und so eine schnelle Ausbreitung resistenter Bakterien ermöglichen.

Heute gibt es eine Vielzahl von Derivaten der beta-Lactam-Antibiotika, darunter mehrere Penicilline (Benzypenicilline, Oralpenicilline, Aminopenicilline, Isoxazylpenicilline, Acylaminopenicilline), des Weiteren Cephalosporine, Monobactame und Carbapeneme, mit in dieser Reihenfolge immer breiter werdendem Wirkspektrum. Die wirksamsten beta-Lactam-Antibiotika sind meist mit größeren chemischen Gruppen derivatisiert, um die beta-Lactamasen sterisch zu behindern. Andererseits bilden sich durch Mutationen in Bakterien immer neue und wirksamere beta-Lactamasen. Die gefürchteten "Extended Spectrum beta-Lactamasen" (ESBL) und die "Carbapenemasen" können ein großes Spektrum an beta-Lactam Antibiotika spalten. Die ESBL sind zunächst durch Punktmutationen an einer beta-Lactamase entstanden. Die Gene für die ESBL und für die Carbapenemasen befinden sich auf Plasmiden, die von Bakterium zu Bakterium horizontal weitergegeben werden können

ESBL tragende Bakterien sind resistent gegen Penicilline, Cephalosporine (Generation 1-4) und gegen Monobactame. Hauptsächlich E. coli und Klebsiellen (Gram-negative Bakterien) tragen ESBL-Gene. Die schnelle Ausbreitung dieser ESBL- und auch der Carbapenem-Resistenzen wird von Mikrobiologen mit größter Sorge betrachtet. Sie ist neben der Methicillin-Resistenz des Staphylococcus aureus (MRSA) einer der sorgenreichsten Brennpunkte der Infektionsforschung, siehe beispielsweise "Rapid Spread of Carbapenem-Resistant Klebsiella pneumoniae in New York City, a New Threat to Our Antibiotic Armamentarium", S. Bratu et al., Arch Intern Med 2005, 165(12), 1430-1435.

Ein Hilfsmittel gegen beta-Lactamasen sind beta-Lactamase-Inhibitoren, welche zusammen mit beta-Lactamen verabreicht werden, um die Wirkung von im Bakterium vorhandenen beta-Lactamasen abzuschwächen. Feste Kombinationspräparate sind beispielsweise Clavulansäure + Amoxicillin, Sulbactam + Ampicillin, Tazobactam + Piperacillin. Es sind nicht alle Kombinationen optimal wirksam. Diese Hilfsmittel sollten jedoch nur nach sorgfältiger Identifizierung der Bakterien und sorgfältiger Resistenzbestimmung angewendet werden, da zu erwarten ist, dass auch dieses Werkzeug sehr schnell wieder stumpf werden wird.

Die Erfindung stellt ein Verfahren bereit, das nur geringe Mengen an Bakterien benötigt, und mit dem eine mikrobielle Resistenz aufgrund einer katalytische Wirkung von mikrobiellen Enzymen auf Antibiotika besonders einfach und schnell gemessen werden kann. Beispielsweise bewirken mikrobielle beta-Lactamasen eine hydrolytische Spaltung des beta-Lactamrings von entsprechenden Antibiotika. Das Verfahren kann die Resistenz der Mikroben in weniger als einer Stunde bestimmen, wobei bevorzugt eine kleine Menge von intakten Mikrobenzellen auf einem massenspektrometrischen Probenträger mit einer dosierten Menge eines Antibiotikums zusammengeführt und dort inkubiert wird. Der Antibiotikum-Abbau aufgrund der katalytischen Wirkung der mikrobiellen Enzyme wird mittels Massenspektrometrie gemessen. Die Inkubationszeit kann dabei überraschenderweise weniger als eine Stunde, in der Regel etwa eine halbe Stunde betragen. Die Inkubation wird überraschenderweise bevorzugt nur bei Raumtemperatur durchgeführt und in feuchter Umgebung vorgenommen, um ein Eintrocknen zu verhindern.

Die Erfindung schließt zudem massenspektrometrische Probenträger (Probenträgerplatte) und Verfahren zu deren Herstellung ein. Eine erfindungsgemäße Probenträgerplatte ist in Abbildung 3 dargestellt; sie ist bevorzugt eben und weist mehrere Probenorte (10) auf, die jeweils bereits mit mindestens einer trockenen Schicht eines Antibiotikums dosiert präpariert sind. Die Probenträgerplatte kann Probenorte mit Schichten verschiedener Antibiotika enthalten, die zur gleichzeitigen Prüfung auf verschiedene Resistenzen einer mikrobiellen Probe verwendet werden können. Es können auch Probenorte Schichten aus Antibiotika mit Inhibitoren für die Reaktionen der mikrobiellen Enzyme vorhanden sein. Die Schichten enthalten auch dosierte Mengen von Referenzsubstanzen enthalten, wobei die Referenzsubstanzen sowohl für eine Re-Kalibrierung der Massenskala, wie auch zur Feststellung quantitativer Änderungen der Massensignale des oder der Antibiotika einschließlich ihrer Abbauprodukte dienen können.

In dem erfindungsgemäßen Verfahren wird die Resistenz der Mikroben durch Zusammenführen und kurzes Inkubieren einer kleinen Menge der Mikroben mit einer festgelegten Menge eines geeigneten Antibiotikums direkt auf einem massenspektrometrischen Probenträger bestimmt, wobei in überraschender Weise bereits nach einer halben Stunde Inkubationszeit bei Raumtemperatur und anschließendem Trocknen und Präparieren einer MALDI-Probe der Abbau des Antibiotikums durch die mikrobiellen Enzyme nachgewiesen werden kann, wenn die MALDI-Probe direkt in einem Massenspektrometer mit einer MALDI-Ionenquelle ohne Entfernung der Mikroben vom Probenträger vermessen wird.

Detailliert aufgelöst in einzelne Verfahrensschritte, stellt sich ein besonders bevorzugtes Verfahren wie folgt dar:

(a) Bereitstellen eines Probenträgers, der auf mindestens einem Probenort eine trockene Schicht mit einer dosierten Menge des Antibiotikums oder des Antibiotikums mit Inhibitor zusammen mit mindestens einer Referenzsubstanz enthält,

(b) Aufbringen der Mikroben auf die trockene Schicht des Antibiotikums und Auftragen einer Flüssigkeit auf diesen Probenort,

(c) Inkubieren auf dem Probenort, insbesondere in einer feuchter Umgebung bei Raumtemperatur,

(d) Trocknen,

(e) Präparation einer Probe für die matrix-unterstützte Laserdesorption und -ionisation (MALDI),

(f) Messung des Massenspektrums in einem Massenspektrometer mit einer MALDI-Ionenquelle, insbesondere mit einem Flugzeitmassenspektrometer, und

(g) Auswertung des Massenspektrums zur Erkennung der Resistenz.

Die Schicht auf dem Probenort enthält beispielsweise etwa 0,1 bis 2 Mikrogramm, vorzugsweise 0,5 Mikrogram Antibiotikum. Sie kann zusätzlich eine Referenzsubstanz in bekannter Menge enthalten. Ähnlich wie bei einer taxonomischen Identifizierung, werden dabei auf jeden Probenort nur etwa $10^3$ bis $10^7$ Mikroben aufgeschmiert. Auf jeden Probenort werden ein bis drei, vorzugsweise etwa zwei Mikroliter Flüssigkeit für das Inkubieren aufgebracht. Verschiedene Probenorte derselben Probenträgerplatte können Schichten mit verschiedenen Antibiotika enthalten. Die Schichten können nicht nur ein Antibiotikum enthalten, sondern auch einen zusätzlichen Inhibitor, der die Wirksamkeit des mikrobiellen Enzyms herabsetzen soll.

Die Mikrobenzellen können manuell aufgeschmiert werden oder aber auch automatisiert aufgebracht werden, beispielsweise durch Aufstempeln. Aus der Druckschrift DE 102012011647 A1 ist zudem ein Verfahren zur massenspektrometrischen Analyse von Mikroben auf einer Nährmitteloberfläche bekannt, bei dem die Mikrobenzellen von der Oberfläche eines flächigen Nährmediums durch direkten Kontakt auf eine Kontaktfläche eines Probenträgers übertragen werden. Der Probenträger kann dabei ein ebener großflächiger Probenträger oder ein stiftförmiger Probenträger, der stirnseitig mit den Mikrobenzellen einer Kolonie in Kontakt gebracht wird, wobei die Kontaktfläche des stiftförmigen Probenträgers so klein ist, dass nur Mikroben einer einzelnen Kolonie auf den stiftförmigen Probenträger übertragen werden. Der stiftförmige Probenträger kann nach der Übertragung von Mikrobenzellen so in eine Adapterplatte eingebracht wird, dass die Stirnseite des stiftförmigen Probenträgers mit der Oberfläche der Adapterplatte im Wesentlichen formschlüssig fluchtet. In der vorliegenden Erfindung kann ein stiftförmiger Probenträger stirnseitig mit einem oder mehreren Antibiotika belegt sein.

Das erfindungsgemäße Verfahren wird im Folgenden anhand eines Beispiels für das breit wirksame Imipenem, einem Carbapenem, dargelegt:

Ein massenspektrometrischer MALDI-Probenträger hat in der Regel eine größere Anzahl (48-386) deutlich markierter Probenorte, die Durchmesser von zwei bis vier Millimeter haben. Besonders günstig sind hydrophile Probenorte in hydrophober Umgebung. In Abbildung 3 ist beispielhaft ein solcher Probenträger mit 96 Probenorten (10) gezeigt; hier in der Größe einer Mikrotiterplatte, es gibt jedoch auch kleinere Ausführungen. Zur Herstellung eines erfindungsgemäßen Probenträger können zumindest einige Probenorte eines Probenträgers mit jeweils zwei Mikroliter einer Lösung von Imipenem (oder eines entsprechenden Antibiotikums) belegt und getrocknet, so dass sich jeweils 0.5 Mikrogramm Imipenem pro Probenort auf dem Probenträger befinden. Nach dem Trocknen wird der Probenträger vor Luftfeuchtigkeit geschützt gelagert, beispielsweise eingeschweißt und mit einem Trockenmittel versehen. Probenträger mit diesen Präparationen können kommerziell angeboten werden, so dass im Labor dieser Präparationsschritt eingespart werden kann.

Bakterien, die als Kolonien auf einer Agar-Platte gewachsen sind, werden anlog zur Schmierpräparation für eine Identifizierung mit einem geeigneten Werkzeug, beispielsweise einem hygienisch sauberen Zahnstocher, manuell auf die trockenen Imipenem-Schichten geschmiert. Dies kann im gleichen Schritt und mit dem gleichen Zahnstocher durchgeführt werden, mit dem eine Probe für eine Identifizierung hergestellt wird. Der Probenort für die Identifizierung weist dabei kein Antibiotikum auf. Wenn alle zu untersuchenden Bakterien auf die Probenorte des Probenträgers aufgetragen sind, werden jeweils zwei Mikroliter eines 10 millimolaren Ammoniumbicarbonat-Puffers (pH 8) aufpipettiert, wobei sich Imipenem und Bakterien miteinander mischen und die Bakterienzellen überwiegend nicht aufgeschlossen werden. Anschließend wird der Probenträger für 30 Minuten bei Raumtemperatur inkubiert, und zwar in einer feucht gehaltenen Box, um ein vorzeitiges Eintrocknen der Probe auf dem Probenort zu verhindern. Nach der Inkubationsphase werden die Proben auf dem Probenträger getrocknet und dann mit Matrixlösung überschichtet, die aber nur sehr wenig Trifluor-Essigsäure enthält, so dass die Bakterienzellen nicht aufgeschlossen werden. Als Matrix wurde $\alpha$-Cyano-4-Hydroxi-Zimtsäure (HCCA) in einer Konzentration von 10 Milligramm pro Milliliter in einer Mischung von Wasser, 50 % Acetonitril und 0,2 % Trifluoressigsäure verwendet. Nach erneutem Trocknen wird von dieser Präparation in einem MALDI-Flugzeitmassenspektrometer in üblicher Weise ein Massenspektrum aufgenommen.

Zur Auswertung der Massenspektren wird in der Regel der Unterscheidungswert $LogRQ_{II}$ gebildet:

$$LogRQ_{II} = Log \frac{\sum Intensitäten\ hydrolysiert}{\sum Intensitäten\ nicht\ hydrolysiert},$$ der in der Regel für beta-Lactamase-positive Mikroben Werte über null, und für Beta-Lactamase-negative Mikroben Werte unter null ergibt.

Die Abbildung 1 zeigt im oberen Teil ein gemessenes MALDI-Massenspektrum, das mit dem erfindungsgemäßen Verfahren und Probenträger von Mikroben gewonnen wurde, die suszeptibel gegenüber Imipenem sind. Die Pfeile markieren dabei Massensignale von nicht abgebautem Imipenem. Der untere Teil der Abbildung 1 zeigt ein gemessenes MALDI-Massenspektrum von resistenten Mikroben, deren Enzyme das Imipenem in nur einer halben Stunde quantitativ hydrolysiert haben, so dass die durch Pfeile angedeuteten Massensignale des Imipenem verschwunden sind. Die enzymatischen Spaltungsreaktionen sind recht schnell: solange sie nicht durch allmählichen Mangel an Substraten behindert werden, liegen sie zwischen etwa einer und hundert Millisekunden pro molekularer Reaktion, mit charakteristischen Unterschieden für die verschiedenen beta-Lactamasen.

Das vorliegende Beispiel mit Imipenem zeigt insofern eine Besonderheit, als dass die enzymatisch hydrolysierten Imipeneme keine Massensignale im MALDI-Massenspektrum aufweisen, so dass es hier notwendig ist, den Abbau der Imipenem-Moleküle zu messen. Dazu ist eine interne Referenzsubstanz erforderlich, die zweckmäßiger Weise bereits der Imipenem-Präparation auf der Probenträgerplatte zugegeben wird. Als interne Referenzsubstanz kann beispielsweise Thiamin verwendet werden. Der senkrechte Strich in den beiden in Abbildung 1 dargestellten Massenspektren gibt die Position der Referenzsubstanz an. Es sei hier angemerkt, dass es auch möglich ist, die Referenzsubstanz später zuzugeben, beispielsweise in der Matrixlösung.

Das Massensignal der internen Referenzsubstanz kann bei der Auswertung der Massenspektren einerseits zu einer Re-Kalibrierung der Massenachse der Massenspektren verwendet werden; andererseits wird es speziell für Imipenem auch zur Berechnung der Abbaurate (Hydrolyserate) herangezogen. Imipenem ist, wie oben dargelegt, insofern ein Spezialfall, als dass die Hydrolyse-Produkte im MALDI-Massenspektrum nicht detektiert werden können, obwohl sie vorhanden sind (mit anderen Arten der Ionisierung, beispielswese Elektrosprüh-Ionisierung (ESI) können die Hydrolyse-Produkte gemessen werden). Um die Reduktion des nicht-hydrolysierten Imipenem-Peaks zumindest erfassen zu können, wird die interne Referenzsubstanz zur Berechnung der Hydrolyserate herangezogen:

$$LogRQ_{RI} = Log \frac{\sum interne\ Referenz}{\sum Intensitäten\ nicht\ hydrolysiert}.$$ Nach Normalisierung der Hydrolysersate auf eine geeignete Negativ- und Positivkontrolle, zeigt eine normalisierte Hydrolyserate norm.$logRQ$ > 0,4 resistente Mikroben an, für suszeptible Mikroben werden Werte norm.$logRQ$ < 0,2 gemessen. Die Abbildung 2 zeigt die Ergebnisse für zwei suszeptible und sechs resistente Mikroben an, gemessen mit einer Imipenem-Belegung auf einem Probenträger nach dem oben angegebenen Herstellungsverfahren.

Die massenspektrometrische Messung im erfindungsgemäßen Verfahren kann im Prinzip mit jedem Massenspektrometer vorgenommen werden, es ist aber besonders günstig, dasselbe MALDI-Flugzeitmassenspektrometer zu benutzen, das derzeit routinemäßig auch für die Identifizierung von Mikroben, insbesondere von Bakterien, eingesetzt wird und das in der Regel im linearen Modus betrieben wird. Die in Abbildung 1 dargestellten Massenspektren sind mit einem MALDI-Flugzeitmassenspektrometer im linearen Modus aufgenommen worden.

In der Regel werden die Mikroben einer Untersuchungsprobe zunächst auf einem Agar-Nährmedium zu Kolonien herangezüchtet, um dann mit einem Werkzeug auf einen Probenort des Probenträgers aufgebracht werden, sowohl für eine Identifizierung wie auch für die Bestimmung von Resistenten. Für eine Identifizierung achtet man üblicherweise darauf, nur Mikroben einer einzigen Kolonie zu entnehmen. Für die Bestimmung der Resistenzen ist es aber oft vorteilhaft, die Mikroben mehrerer Kolonien, mindestens etwa fünf, zu mischen, um auch bei Mischresistenzen zu brauchbaren Ergebnissen zu kommen.

Eine andere wichtige Art der Züchtung ist die Blutkultur, die bei einer diagnostizierten oder vermuteten Sepsis angewendet wird. Der Patient wird in der Regel sofort mit dem sehr breitbandigen Imipenem behandelt, da Sepsen außerordentlich gefährlich sind und eine hohe Sterberate zeigen. Nach einigen Stunden Kultivierung werden die Blutzellen durch geeignete Maßnahmen zerstört und die Mikroben der Sepsis können mit vorsichtigem Zentrifugieren oder Filtrieren so abgesetzt werden, dass die Mikroben nicht geschädigt werden. Ein Teil der Mikrobenzellen kann für eine taxonomische Identifizierung, der andere Teil für den erfindungsgemäßen Resistenztest gegen Imipenem verwendet werden. Liegt eine solche Resistenz gegen Imipenem vor, so kann mit Kombinationspräparaten weiterbehandelt werden, die gezielter ausgewählt werden können, da die Art der Mikroben durch die gleichzeitige Identifizierung bekannt ist.

Im Allgemeinen interessiert nicht nur die Resistenz gegen ein einziges Antibiotikum. Das erfindungsgemäße Verfahren erlaubt es, auch Multiplex-Resistenztests mit Einbringen mehrerer verschiedenartiger Antibiotika zu entwickeln, wobei die verschiedenen Antibiotika auf verschiedene Probenorte eines Probenträgers, aber auch auf einen einzelnen Probenort gelegt werden können. Es können Probenträger hergestellt werden, die bereits Reihen verschiedener Antibiotika enthalten, gegebenenfalls mit Referenzsubstanzen. Es können in zusätzlichen Probenorten auch Antibiotika mit Inhibitoren (Kombinationspräparate) zusammen aufgebracht werden. In dem Resistenztest mit verschiedenen Antibiotika an verschiedenen Probenorten können die zu testenden Mikroben mit demselben Werkzeug auf die verschiedenen Probenorte verteilt werden. Das Pipettieren des Puffers, das Inkubieren, Präparieren und die massenspektrometrische Messung erfolgen wie im obigen Beispiel dargestellt. Die Auswertung kann mit einem Programm erfolgen, das auch die Messung im Massenspektrometer steuert und die leicht verschiedenen Parameter für die Auswertung der Massenspektren der verschiedenen Probenorte kennt. Es wird so die Resistenz gegenüber verschiedenen Antibiotika in einem Durchgang gemessen, der kaum länger dauert, als die Bestimmung der Resistenz gegenüber einem Antibiotikum.

Es gibt auch Fälle von Mischproben, in denen man weniger an einer Identifizierung als vielmehr an einer sofortigen Resistenzbestimmung interessiert ist. So ist es außerordentlich wichtig zu wissen, ob in einem Abstrich der Nasenschleimhaut oder im Nasensekret resistente Keime enthalten sind. Anhand eines Abstrichs der Nasen- oder Mundschleimhaut wird nämlich in der Regel untersucht, ob ein Patient bei Aufnahme in ein Krankenhaus oder ein Mitarbeiter eines Krankenhauses Träger einer mikrobiellen Resistenz ist. Ein solcher Abstrich oder Nasensekret kann als mikrobielle Probe direkt auf entsprechend vorpräparierte Probenorte eines Probenträgers aufgebracht werden, wobei aufgrund der Vielzahl von derartigen Resistenzbestimmungen in einem Krankenhaus ein Probenort bevorzugt mehr als ein Antibiotikum aufweist. Ähnliches gilt auch für andere Arten von Infektionen, beispielsweise in eitrigen Wundherden.

Die Erfindung ist dabei nicht auf die aufgeführten Ausführungsformen oder die Beispiele beschränkt. So kann beispielsweise die Ionisierung auch mittels DESI (Desorption Electrospray Ionization) von einem ebenen Probenträger oder auch direkt von der Oberfläche eines flächigen Nährmediums erfolgen, das als Probenträger dient und auf das eine Flüssigkeit mit dem Antibiotikum aufgebracht wird. Neben der Ionisierung durch MALDI ist beispielsweise auch eine Laserdesorption von einem ebenen Probenträger mit nachfolgender chemischer Ionisierung ist möglich.

**Patentansprüche**

1. Verfahren für die Bestimmung der Resistenz einer mikrobiellen Probe auf ein Antibiotikum oder auf ein Antibiotikum mit Inhibitor, wobei die mikrobielle Probe und das Antibiotikum oder die mikrobielle Probe und das Antibiotikum mit dem Inhibitor in einem Flüssigkeitsvolumen auf einem Proben ort eines massenspektrometrischen Probenträgers zusammengeführt und inkubiert werden und durch eine massenspektrometrische Messung am Probenort untersucht wird, ob das Antibiotikum enzymatisch verändert wird, **dadurch gekennzeichnet, dass**

   - der Probenort vor dem Zusammenführen mit einer dosierten Menge des Antibiotikum oder des Antibiotikum mit Inhibitor zusammen mit mindestens einer Referenzsubstanz in Form einer trockenen Schicht belegt ist, und
- eine Abnahme des Antibiotikums aus dem Vergleich mit dem Massensignal der mindestens einen Referenzsubstanz ermittelt wird und/oder die mindestens eine Referenzsubstanz zur Feststellung der quantitativen Änderung der Abbauprodukte des Antibiotikums verwendet wird.

2. Verfahren nach Anspruch 1, **dadurch gekennzeichnet, dass** die mikrobielle Probe intakte Mikrobenzellen aufweist.

3. Verfahren nach Anspruch 2, **dadurch gekennzeichnet, dass** die Inkubationszeit in dem Flüssigkeitsvolumen weniger als eine Stunde beträgt.

4. Verfahren nach einem der Ansprüche 2 oder 3, **dadurch gekennzeichnet, dass** die Inkubation in dem Flüssigkeitsvolumen bei Raumtemperatur vorgenommen wird.

5. Verfahren nach Anspruch 2 bis 4, **dadurch gekennzeichnet, dass** auf den Probenort jeweils zwischen $10^3$ bis $10^7$ Mikrobenzellen aufgebracht werden.

6. Verfahren nach Anspruch 2 bis 5, **dadurch gekennzeichnet, dass** am Probenort eine Probe für die matrix-unterstützte Laserdesorption und -ionisation präpariert wird, ohne die Mikroben aufzuschließen.

7. Verfahren nach einem der Ansprüche 2 bis 6, mit den Schritten:

(a) Bereitstellen eines massenspektrometrischen Probenträgers, der auf mindestens einem Probenort eine trockene Schicht mit einer dosierten Menge des Antibiotikums oder des Antibiotikums mit Inhibitor zusammen mit der mindestens einen Referenzsubstanz enthält,
(b) Aufbringen der intakten Mikrobenzellen auf die trockene Schicht des Antibiotikums oder des Antibiotikums mit Inhibitor zusammen mit der mindestens einen Referenzsubstanz und Auftragen der Flüssigkeit,
(c) Inkubieren auf dem Probenort,
(d) Trocknen,
(e) Präparation einer Probe für die matrix-unterstützte Laserdesorption und -ionisation (MALDI),
(f) Messung des Massenspektrums in einem Massenspektrometer mit einer MALDI-Ionenquelle, und
(g) Auswertung des Massenspektrums zur Erkennung der Resistenz.

8. Verfahren nach Anspruch 7, **dadurch gekennzeichnet, dass** die Schicht auf dem Probenort etwa 0,1 bis 2 Mikrogramm, vorzugsweise 0,5 Mikrogram Antibiotikum enthält.

9. Verfahren nach Anspruch 1, **dadurch gekennzeichnet, dass** am Probenort eine Probe für die matrix-unterstützte Laserdesorption und -ionisation präpariert wird, wodurch die Mikroben in der mikrobiellen Probe aufgeschlossen werden, oder die mikrobielle Probe ein Zelllysat ist und dass in der massenspektrometrische Messung auch Massensignale von mikrobiellen Proteinen aufgenommen werden, mit denen die Mikroben taxonomisch identifiziert und/oder zusätzlich hinsichtlich ihres Resistenzverhaltens charakterisiert werden.

10. Verfahren nach Anspruch 1, **dadurch gekennzeichnet, dass** die mikrobielle Probe aufgeschlossene Mikroben aufweist.

11. Verfahren nach einem der Ansprüche 1 bis 10, **dadurch gekennzeichnet, dass** das Flüssigkeitsvolumen kleiner als zehn Mikroliter ist.

12. Verfahren nach einem der Ansprüche 1 bis 11, **dadurch gekennzeichnet, dass** die mikrobielle Probe mit mehr als einem Antibiotikum im Flüssigkeitsvolumen am Probenort zusammengeführt wird.

13. Verfahren nach Anspruch 12, **dadurch gekennzeichnet, dass** die mikrobielle Probe ein Abstrich ist.

14. Verfahren nach Anspruch 1, **dadurch gekennzeichnet, dass** das Antibiotikum Imipenem ist und Thiamin die Referenzsubstanz ist.

15. Verfahren nach einem der Ansprüche 1 bis 14, **dadurch gekennzeichnet, dass** der massenspektrometrische Probenträger nach dem Zusammenführen in einer feuchten Umgebung gehalten wird oder dem Flüssigkeitsvolumen auf dem Probenort nach dem Zusammenführen zusätzlich Flüssigkeit zugeführt wird, um ein Eintrocknen des Flüssigkeitsvolums für eine vorgegebene Reaktionszeit zu verhindern.

16. Probenträger für eine massenspektrometrische Bestimmung der Resistenz einer mikrobiellen Probe auf ein Antibiotikum oder auf ein Antibiotikum mit Inhibitor, **dadurch gekennzeichnet, dass** der Probenträger eine Vielzahl von Probenorten aufweist und dass mindestens ein Probenort mit einer dosierten Menge des Antibiotikums oder des Antibiotikums mit Inhibitor zusammen mit mindestens einer Referenzsubstanz in Form einer trockenen Schicht belegt ist.

**Claims**

1. Method for determining the resistance of a microbial sample on an antibiotic or on an antibiotic with inhibitor, wherein the microbial sample and the antibiotic or the microbial sample and the antibiotic with the inhibitor are combined and incubated in a volume of liquid at a test site of a mass spectrometric sample support and analyzed by means of a mass spectrometric measurement at the test site to ascertain whether the antibiotic is enzymatically modified, **wherein**

   - the test site is coated with a dosed quantity of the antibiotic or the antibiotic with inhibitor together with at least one reference substance in the form of a dry layer before they are combined, and
   - it is determined that there is a decrease in the antibiotic by comparison with the mass signal of the (at least one) reference substance and/or the (at least one) reference substance is used to determine the quantitative change to the degradation products of the antibiotic.

2. The method according to Claim 1, wherein the microbial sample has intact microbial cells.

3. The method according to Claim 2, wherein the incubation period in the volume of liquid is less than an hour.

4. The method according to one of Claims 2 or 3, wherein the incubation in the volume of liquid is carried out at room temperature.

5. The method according to Claims 2 to 4, wherein between $10^3$ and $10^7$ microbial cells are applied to each test site.

6. The method according to Claims 2 to 5, wherein a sample for matrix-assisted laser desorption and ionization is prepared at the test site without the microbes being digested.

7. The method according to one of Claims 2 to 6 with the steps:

   (a) Provision of a mass spectrometric sample support containing a dry layer with a dosed quantity of the antibiotic or the antibiotic with inhibitor together with the (at least one) reference substance at one or more test sites,
   (b) Application of the intact microbe cells onto the dry layer of the antibiotic or the antibiotic with inhibitor together with the (at least one) reference substance and application of the liquid,
   (c) Incubation at the test site,
   (d) Drying,
   (e) Preparation of a sample for the matrix-assisted laser desorption and ionization (MALDI),
   (f) Measurement of the mass spectrum in a mass spectrometer with a MALDI ion source, and
   (g) Evaluation of the mass spectrum to identify the resistance.

8. The method according to Claim 7, wherein the layer on the test site contains around 0.1 to 2 micrograms, preferably 0.5 micrograms, of antibiotic.

9. The method according to Claim 1, wherein a sample for matrix-assisted laser desorption and ionization is prepared at the test site, whereby the microbes in the microbial sample are digested, or the microbial sample is a cell lysate, and mass signals of microbial proteins are also acquired in the mass spectrometric measurement, said mass signals allowing the microbes to be identified taxonomically and/or characterized additionally in respect of their resistance behavior.

10. The method according to Claim 1, wherein the microbial sample contains digested microbes.

**11.** The method according to one of Claims 1 to 10, wherein the volume of liquid is less than ten microliters.

**12.** The method according to one of Claims 1 to 11, wherein the microbial sample is combined with more than one antibiotic in the volume of liquid at the test site.

**13.** The method according to Claim 12, wherein the microbial sample is a swab.

**14.** The method according to Claim 1, wherein the antibiotic is imipenem and the reference substance is thiamine.

**15.** The method according to one of Claims 1 to 14, wherein the mass spectrometric sample support is kept in a humid environment after everything has been combined, or additional liquid is added to the volume of liquid at the test site after everything has been combined in order to prevent the volume of liquid from drying up for a specified reaction time.

**16.** Sample support for a mass spectrometric determination of the resistance of a microbial sample on an antibiotic or on an antibiotic with inhibitor, wherein the sample support has a large number of test sites and at least one test site is coated with a dosed quantity of the antibiotic or the antibiotic with inhibitor together with at least one reference substance in the form of a dry layer.

**Revendications**

**1.** Méthode utilisée pour déterminer la résistance d'un échantillon microbien à un antibiotique ou à un antibiotique avec inhibiteur, l'échantillon microbien et l'antibiotique ou l'échantillon microbien et l'antibiotique avec inhibiteur étant concentrés et incubés dans un volume de liquide sur le site d'un porte-échantillon par spectrométrie de masse et un examen étant effectué sur le site au moyen d'une mesure par spectrométrie de masse pour savoir si l'antibiotique subit une modification enzymatique, **caractérisée en ce que**

- avant la concentration, le site est occupé sous forme de couche sèche par une quantité dosée de l'antibiotique ou de l'antibiotique avec inhibiteur, à laquelle est ajoutée au moins une substance de référence, et
- une réduction de l'antibiotique est calculée à partir de la comparaison avec le signal de masse d'au moins une substance de référence, et/ou au moins une substance de référence est utilisée pour constater la modification quantitative des produits de dégradation de l'antibiotique.

**2.** Méthode selon la revendication 1, **caractérisée en ce que** l'échantillon microbien présente des cellules microbiennes intactes.

**3.** Méthode selon la revendication 2, **caractérisée en ce que** la période d'incubation dans le volume de liquide dure moins d'une heure.

**4.** Méthode selon l'une des revendications 2 ou 3, **caractérisée en ce que** l'incubation est effectuée dans le volume de liquide à température ambiante.

**5.** Méthode selon les revendications 2 à 4, **caractérisée en ce qu'**entre $10^3$ à $10^7$ cellules microbiennes sont appliquées sur chaque site.

**6.** Méthode selon les revendications 2 à 5, **caractérisée en ce qu'**un échantillon est préparé sur le site pour la désorption-ionisation laser assistée par matrice, sans désagréger les microbes.

**7.** Méthode selon l'une des revendications 2 à 6, avec les étapes suivantes :

(a) Mise à disposition d'un porte-échantillon par spectrométrie de masse, qui contient au moins sur un site une couche sèche avec une quantité dosée de l'antibiotique ou de l'antibiotique avec inhibiteur, à laquelle est ajoutée au moins une substance de référence ;
(b) Application des cellules microbiennes intactes sur la couche sèche de l'antibiotique ou de l'antibiotique avec inhibiteur, à laquelle est ajoutée au moins une substance de référence, et application du liquide ;
(c) Incubation sur le site ;
(d) Séchage ;
(e) Préparation d'un échantillon pour la désorption-ionisation laser assistée par matrice (MALDI) ;

(f) Mesure du spectre de masse dans un spectromètre de masse avec une source d'ions MALDI; et

(g) Analyse du spectre de masse aux fins d'identification de la résistance.

8. Méthode selon la revendication 7, **caractérisée en ce que** la couche sur le site contient environ 0,1 à 2 microgrammes, de préférence 0,5 microgramme d'antibiotique.

9. Méthode selon la revendication 1, **caractérisée en ce qu'**un échantillon est préparé sur le site pour la désorption-ionisation laser assistée par matrice, entraînant la désagrégation des microbes dans l'échantillon microbien, ou **en ce que** l'échantillon microbien est un lysat de cellules et que la mesure de spectrométrie de masse comprend également l'enregistrement de signaux de masse provenant de protéines microbiennes, avec lesquels on effectue une identification taxonomique des microbes et/ou, en plus, une caractérisation des microbes en fonction de leur résistance.

10. Méthode selon la revendication 1, **caractérisée en ce que** l'échantillon microbien présente des microbes désagrégés.

11. Méthode selon l'une des revendications 1 à 10, **caractérisée en ce que** le volume de liquide est inférieur à dix microlitres.

12. Méthode selon l'une des revendications 1 à 11, **caractérisée en ce que** l'échantillon microbien est concentré avec plus d'un antibiotique dans le volume de liquide sur le site.

13. Méthode selon la revendication 12, **caractérisée en ce que** l'échantillon microbien est un prélèvement.

14. Méthode selon la revendication 1, **caractérisée en ce que** l'antibiotique est de l'imipénem et la substance de référence est de la thiamine.

15. Méthode selon l'une des revendications 1 à 14, **caractérisée en ce que** le porte-échantillon par spectrométrie de masse est maintenu après la concentration dans un milieu humide ou que du liquide est ajouté au volume de liquide sur le site après la concentration, afin d'empêcher un dessèchement du volume de liquide pendant un temps de réaction prédéfini.

16. Porte-échantillon utilisé pour déterminer par la spectrométrie de masse la résistance d'un échantillon microbien à un antibiotique ou à un antibiotique avec inhibiteur, **caractérisé en ce que** le porte-échantillon présente un grand nombre de sites et qu'au moins un site est occupé sous forme de couche sèche par une quantité dosée de l'antibiotique ou de l'antibiotique avec inhibiteur, à laquelle est ajoutée au moins une substance de référence.

Abbildung 1

Abbildung 2

Abbildung 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2011154517 A1, M. Kostrzewa, K. Michelmann, K. Sparbier
- DE 102010023452 B4
- US 20130095511 A1
- WO 2012023845 A1, Luider
- DE 102012011647 A1

**In der Beschreibung aufgeführte Nicht-Patentliteratur**

- **S. BRATU et al.** Rapid Spread of Carbapenem-Resistant Klebsiella pneumoniae in New York City, a New Threat to Our Antibiotic Armamentarium. *Arch Intern Med,* 2005, vol. 165 (12), 1430-1435